# EUROPEAN PATENT SPECIFICATION

(11) **EP 4 370 035 B1**
(45) Date of publication and mention of the grant of the patent: **28.10.2026**
(21) Application number: 22825736.6
(22) Date of filing: 15.06.2022
(51) Int. Cl.: A61B 8/00, G16H 10/00, A61B 8/08

(54) **METHODS AND APPARATUSES FOR GUIDING A USER TO COLLECT ULTRASOUND IMAGES**
VERFAHREN UND VORRICHTUNGEN ZUR FÜHRUNG EINES BENUTZERS ZUR SAMMLUNG VON ULTRASCHALLBILDERN
PROCÉDÉS ET APPAREILS PERMETTANT DE GUIDER UN UTILISATEUR POUR COLLECTER DES IMAGES ULTRASONORES

(30) Priority: 16.06.2021 US 202163211517 P
(43) Date of publication of application: 22.05.2024
(73) Proprietor: BFLY Operations, Inc., Burlington, MA 01803 (US); Daks, Alon, Scarsdale, NY 10583 (US); Howell, Audrey, Guilford, CT 06437 (US)
(72) Inventor: SAMANGOUEI, Pouya, San Francisco, CA 94114 (US); SHIN, Brian, New York, NY 10128 (US); MANI, Jai, Brooklyn, NY 11201-5457 (US); DAKS, Alon, Scarsdale, NY 10583 (US); HOWELL, Audrey, Guilford, CT 06437 (US)
(74) Representative: Carroll, Christopher P.
(86) International application number: PCT/US2022/033590
(87) International publication number: WO 2022/266197

(56) References cited:
- WO-A1-2021/014773
- WO-A1-2021/014773
- US-A1- 2010 036 252
- US-A1- 2017 296 152
- US-A1- 2019 261 957
- US-A1- 2020 170 619
- US-A1- 2020 214 674
- US-A1- 2021 052 249
- US-A1- 2022 104 791
- US-B2- 10 588 606
- US-B2- 10 893 850

## Description

### FIELD

Generally, the aspects of the technology described herein relate to collection of ultrasound data. Some aspects relate to guiding a user to collect ultrasound images.

### BACKGROUND

Ultrasound devices may be used to perform diagnostic imaging and/or treatment, using sound waves with frequencies that are higher than those audible to humans. Ultrasound imaging may be used to see internal soft tissue body structures. When pulses of ultrasound are transmitted into tissue, sound waves of different amplitudes may be reflected back towards the probe at different tissue interfaces. These reflected sound waves may then be recorded and displayed as an image to the operator. The strength (amplitude) of the sound signal and the time it takes for the wave to travel through the body may provide information used to produce the ultrasound image. Many different types of images can be formed using ultrasound devices. For example, images can be generated that show two-dimensional cross-sections of tissue, blood flow, motion of tissue over time, the location of blood, the presence of specific molecules, the stiffness of tissue, or the anatomy of a three-dimensional region. WO2021/014773A1 describes an ultrasonic diagnostic device with: a gel layer thickness calculation unit for calculating the thickness of a gel layer by analyzing an ultrasonic image; a tissue recognition unit for recognizing the tissue of a test subject by analyzing the ultrasonic image; and a compression state determination unit for determining, when the calculated thickness of the gel layer is equal to or less than a thickness threshold value, the compression state of an ultrasound probe against the body surface of the test subject, on the basis of shape change of the tissue recognized by the tissue recognition unit.

### BRIEF DESCRIPTION OF THE DRAWINGS

Various aspects and embodiments will be described with reference to the following exemplary and non-limiting figures. It should be appreciated that the figures are not necessarily drawn to scale. Items appearing in multiple figures are indicated by the same or a similar reference number in all the figures in which they appear.
FIG. 1 illustrates an example process 100 for guiding a user to collect one or more ultrasound images, in accordance with certain embodiments described herein.
FIGs. 2-4 illustrate example graphical user interfaces for guiding a user to collect one or more ultrasound images, in accordance with certain embodiments described herein.
FIG. 5 illustrates a schematic block diagram of an example ultrasound system upon which various aspects of the technology described herein may be practiced.

### DETAILED DESCRIPTION

The invention is set out in the appended set of claims. Conventional ultrasound systems are large, complex, and expensive systems that are typically only purchased by large medical facilities with significant financial resources. Recently, less expensive and less complex ultrasound imaging devices have been introduced. Such devices may include ultrasonic transducers monolithically integrated onto a single semiconductor die to form a monolithic ultrasound device. Aspects of such ultrasound-on-a chip devices are described in U.S. Patent Application No. 15/415,434 titled "UNIVERSAL ULTRASOUND DEVICE AND RELATED APPARATUS AND METHODS," filed on January 25, 2017 (and assigned to the assignee of the instant application) and published as U.S. Pat. Pub. No. US2017/0360397 Al. The reduced cost and increased portability of these new ultrasound devices may make them significantly more accessible to the general public than conventional ultrasound devices. Patients with chronic conditions may benefit from use of such ultrasound devices in their homes. As one example, patients with urinary incontinence may benefit from using an ultrasound device at home to determine when their bladder is full.

Steps necessary to capture clinically relevant ultrasound images may include applying sufficient gel to the ultrasound device, positioning the ultrasound device on the subject, properly positioning the ultrasound device on the subject such that a clinically usable ultrasound image of a particular anatomical feature and/or from a particular anatomical view can be collected, and holding the ultrasound device steady while ultrasound images of the particular anatomical feature (e.g., the heart, bladder, lungs, etc.) and/or from the particular anatomical view are collected. Conventional guidance systems may rely on a novice at-home user determining when a particular step has been completed. However, this may be challenging, as a user may not determine correctly when a particular step has been completed, and certain problems may occur during the guidance which require corrective action, but which a user may not readily notice. Such problems may include the ultrasound device no longer contacting the subject, the anatomical feature or anatomical view of interest no longer being present in ultrasound images collected by the ultrasound device, and their not being sufficient ultrasound coupling medium (referred to herein for simplicity as "gel") applied to the ultrasound device (e.g., because the user did not put any gel on the ultrasound device, or the gel wiped off on the subject).

The inventors have developed technology to address these problems. The technology may include providing various instructions to the user (where any given instruction, and sometimes each instruction, may be one of multiple steps needed to prepare the ultrasound device for collecting clinically usable ultrasound images), automatically detecting when the user has completed a step based on ultrasound images collected by the ultrasound device, and automatically transitioning to providing an instruction for a following step. The steps may include the following: applying sufficient gel to the ultrasound device; positioning the ultrasound device on the subject; properly positioning the ultrasound device on the subject such that a clinically usable ultrasound image of a particular anatomical feature and/or from a particular anatomical view can be collected; and holding the ultrasound device steady while ultrasound images of the particular anatomical feature (e.g., the heart, bladder, lungs, etc.) and/or from the particular anatomical view are collected. The processing device may automatically move through each of the above steps one-by-one, or if the user has already completed one or more of the steps prior to beginning the guidance, then the processing device may skip one or more of the steps.

To determine when a user has completed a step, the processing device may use one or more statistical models trained to determine a state of the ultrasound device based on ultrasound images collected by the ultrasound device. The states detected by such a statistical model may include whether the ultrasound device is on the subject or not (the state of the ultrasound device not being on the subject being referred to herein as "in the air" regardless of where the ultrasound probe is), whether the ultrasound device has sufficient gel or not, and whether the ultrasound device is properly positioned for capturing clinically usable ultrasound images or not.

It should be appreciated that the embodiments described herein may be implemented in any of numerous ways. Examples of specific implementations are provided below for illustrative purposes only. It should be appreciated that these embodiments and the features/capabilities provided may be used individually, all together, or in any combination of two or more, as aspects of the technology described herein are not limited in this respect.

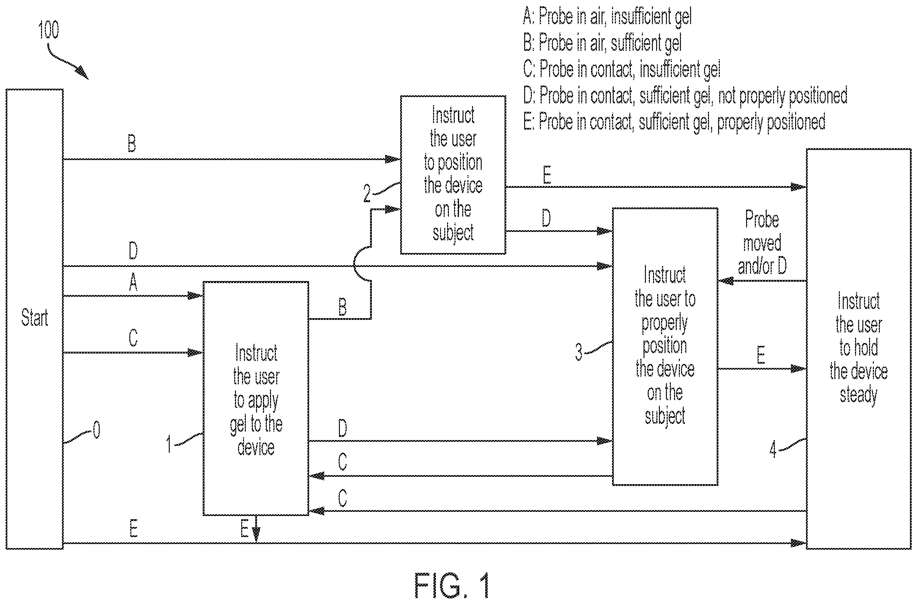

FIG. 1 illustrates an example process 100 for guiding a user to collect one or more ultrasound images, in accordance with certain embodiments described herein. The process 100 is performed by a processing device. The processing device may be, for example, a mobile phone, tablet, or laptop in operative communication with an ultrasound device. The processing device and the ultrasound device may communicate over a wired communication link (e.g., over Ethernet, a Universal Serial Bus (USB) cable or a Lightning cable) and/or over a wireless communication link (e.g., over a BLUETOOTH, WiFi, or ZIGBEE wireless communication link).

Generally, the process 100 may include guiding a user of the ultrasound device (who may be a novice) to collect a clinically usable ultrasound image of a particular anatomical feature (e.g., the heart, bladder, lungs, etc.) and/or from a particular anatomical view (e.g., parasternal long-axis view of the heart, apical four-chamber view of the heart). The guidance may include the processing device providing various instructions to the user (e.g., by displaying the instructions on its display screen and/or outputting the instructions from its speaker). In some embodiments, each instruction may be one of multiple steps needed to prepare the ultrasound device for collecting clinically usable ultrasound images. The processing device may automatically detect when the user has completed a step and move on to providing an instruction for a following step. The steps may include the following:
1. Applying sufficient gel to the ultrasound device;
2. Positioning the ultrasound device on the subject;
3. Properly positioning the ultrasound device on the subject such that a clinically usable ultrasound image of the particular anatomical feature and/or from the particular anatomical view can be collected; and
4. Holding the ultrasound device steady while ultrasound images of the particular anatomical feature (e.g., the heart, bladder, lungs, etc.) and/or from the particular anatomical view are collected;

The processing device may automatically move through each of the above steps one-by-one, or if the user has already completed one or more of the steps prior to beginning the guidance, then the processing device may skip one or more of the steps.

During one or more acts of the process 100, the processing device may configure the ultrasound device to collect ultrasound images (e.g., at a rate of at least 5Hz, at least 10 Hz, at least 20Hz, at a rate between 5 and 60 Hz, and/or at a rate of more than 20 Hz). As referred to herein, an ultrasound device collecting ultrasound images may mean that the ultrasound device collects raw acoustical data, generates scan lines from the raw acoustical data, generates ultrasound images from the scan lines, and the processing device receives the ultrasound images from the scan lines; that the ultrasound device collects raw acoustical data and generates scan lines from the raw acoustical data, and the processing device receives the scan lines from the ultrasound device and generates ultrasound images from the scan lines; that the ultrasound device collects raw acoustical data and the processing device receives the raw acoustical data, generates scan lines from the raw acoustical data, and generates ultrasound images from the scan lines; or any other means by which the ultrasound device and the processing device may, in combination, generate ultrasound images from raw acoustical data.

The processing device may determine a state of the ultrasound device based on one or more collected ultrasound images (e.g., the most recently collected ultrasound image, or a certain number of the most recently collected ultrasound images). States of the ultrasound device may include the following:
A. The ultrasound device is in the air and has insufficient gel
B. The ultrasound device is in the air and has sufficient gel
C. The ultrasound device is in contact with the subject and has insufficient gel
D. The ultrasound device is in contact with the subject, has sufficient gel, and is not properly positioned; and
E. The ultrasound device is in contact with the subject, has sufficient gel, and is properly positioned.

Based at least in part on the state of the ultrasound device as determined by the processing device from one or more collected ultrasound images (e.g., the most recently collected ultrasound image, or a certain number of the most recently collected ultrasound images), the processing device may provide a particular instruction. For example, the processing device may determine whether sufficient gel has been applied to the ultrasound device. Based on determining that sufficient gel has not been applied to the ultrasound device (e.g., the ultrasound device is in state A or C), the processing device may instruct the user to apply sufficient gel to the ultrasound device. As another example, the processing device may determine whether the ultrasound device has been positioned on the subject. Based on determining that the processing device has not been positioned on the subject (e.g., the ultrasound device is in state B), the processing device may instruct the user to position the ultrasound device on the subject. As another example, the processing device may determine whether the ultrasound device has been properly positioned on the subject in order to capture clinically usable ultrasound images of a particular anatomical feature and/or from a particular anatomical view. Based on determining that the ultrasound device has not been properly positioned on the subject in order to capture clinically usable ultrasound images of a particular anatomical feature and/or from a particular anatomical view (e.g., the ultrasound device is in state D), the processing device may instruct the user to properly position the ultrasound device on the subject in order to capture the clinically usable ultrasound images of the particular anatomical feature and/or from the particular anatomical view. As will be described further below, if the state of the ultrasound device changes (e.g., based on the user successfully following a currently provided instruction), the processing device may automatically transition from providing the current instruction to providing a new instruction.

In some embodiments, the processing device may use a statistical model to determine the state of the ultrasound device based on one or more ultrasound images collected by the ultrasound device (e.g., the most recently collected ultrasound image, or a certain number of the most recently collected ultrasound images). Accordingly, when making the determinations described above and/or when providing the instructions described above, the ultrasound device may be collecting ultrasound images, and the processing device may receive these ultrasound images and use them to make the determinations. The statistical model may be stored on the processing device, or may be stored on another device (e.g., a server) and the processing device may access the statistical model on that other device. The statistical model may be trained on multiple ultrasound images, each of which was collected with and labeled as having been collected when the ultrasound device was in a particular state (e.g., one of the states described above). Thus, the training ultrasound images may include ultrasound images collected and labeled as having being collected when the ultrasound device was in the air and did not have sufficient gel; when the ultrasound device was in the air and had sufficient gel; when the ultrasound device was in contact with the subject and did not have sufficient gel; when the ultrasound device was in contact with the subject, had sufficient gel, and was not properly positioned; and when the ultrasound device was in contact with the subject, had sufficient gel, and was properly positioned. Based on the training, the statistical model may determine, based on one or more inputted ultrasound images (e.g., the most recently collected ultrasound image, or a certain number of the most recently collected ultrasound images), which state the ultrasound device was in when it collected the one or more ultrasound images. Any of the determinations about the current state of the ultrasound device made by the processing device may be made using a statistical model and based on ultrasound images recently collected by the ultrasound device, as described above.

It should be appreciated that different statistical models may be used based on the goal of the ultrasound imaging session - in other words, based on which particular anatomical feature and/or which particular anatomical view is desired to be present in the collected ultrasound image(s). For example, if the goal of the ultrasound imaging session is to collect clinically usable ultrasound images of the bladder, then the processing device may use a statistical model in which those training ultrasound images labeled as having been collected when the ultrasound device was properly positioned were collected when the ultrasound device was collecting ultrasound images of the bladder. If the goal of the ultrasound imaging session is to collect clinically usable ultrasound images of the lungs, then the processing device may use a statistical model in which those training ultrasound images labeled as having been collected when the ultrasound device was properly positioned were collected when the ultrasound device was collecting ultrasound images of the lungs. The user may select an option corresponding to the goal of the imaging session (e.g., prior to steps 1, 2, and/or 3 of the process 100) or the processing device may automatically select the goal of the imaging session (e.g., as part of an automatic workflow). In some embodiments, multiple statistical models may be used. For example, one statistical model may be used to determine whether the ultrasound device is in states A, B, or C, while another statistical model may be used to determine whether the ultrasound device is in states D or E. In such embodiments, the former statistical model may be used regardless of the goal of the ultrasound imaging session.

The process 100 begins at step 0. If the processing device determines at step 0 that the ultrasound device is in the air and has insufficient gel (state A), or that the ultrasound device is in contact with the subject and has insufficient gel (state C), the process 100 proceeds to step 1. If the processing determines that the ultrasound device is in the air and has sufficient gel (state B), the process 100 proceeds to step 2. If the processing determines that the ultrasound device is in contact with the subject, has sufficient gel, and is not properly positioned on the subject (state D), the process 100 proceeds to step 3. If the processing determines that the ultrasound device is in contact with the subject, has sufficient gel, and is properly positioned on the subject (state E), the process 100 proceeds to step 4.

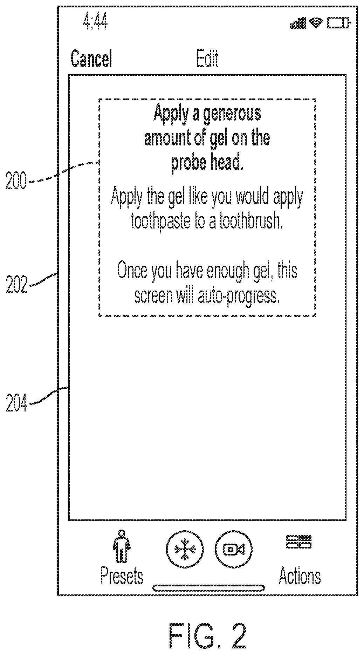

At step 1, the processing device instructs the user to apply sufficient gel to the ultrasound device. Example instructions are described further with reference to FIG. 2. The process 100 may remain at step 1 until the processing device determines that the ultrasound device now has sufficient gel. In particular, if the processing device determines that the ultrasound device is in air and now has sufficient gel (state B), the process 100 proceeds to step 2. If the processing device determines that that the ultrasound device is in contact with the subject, now has sufficient gel, and is not properly positioned (state D), the process 100 proceeds to step 3. If the processing device determines that the ultrasound device is in contact with the subject, now has sufficient gel, and is properly positioned (state E), the process 100 proceeds to step 4.

In some embodiments, at step 1, the processing device may provide feedback as to how much gel has been applied to the ultrasound device and/or how much gel still needs to be applied to the ultrasound device. Thus, the processing device may indicate one or multiple levels of gel that has been applied to the ultrasound device and/or one of multiple levels of gel that still needs to be applied to the ultrasound device. For example, the levels may be none, low, medium, and high, where high is considered sufficient. In such embodiments, instead of or in addition to determining whether the ultrasound device is in state A or state B, the statistical model may be trained to determine whether the ultrasound device has various amounts of gel applied to it. The statistical model may be trained on ultrasound images collected with and labeled as having been collected with no gel, low amounts of gel, medium amounts of gel, and high amounts of gel (or any other number of levels) applied to the ultrasound device. The processing device may provide feedback, for example, by displaying a progress bar.

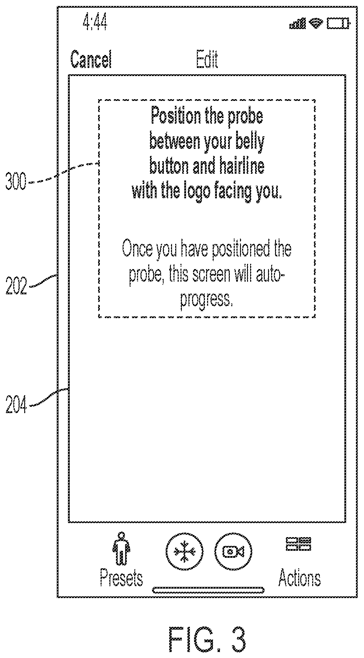

At step 2, the processing device instructs the user to position the ultrasound device on the subject. Example instructions are described further with reference to FIG. 3. The process 100 may remain at step 2 until the processing device determines that the ultrasound device now is in contact with the subject. In particular, if the processing device determines that that the ultrasound device is now in contact with the subject, has sufficient gel, and is not properly positioned (state D), the process 100 proceeds to step 3. If the processing device determines that the ultrasound device is now in contact with the subject, has sufficient gel, and is properly positioned (state E), the process 100 proceeds to step 4.

At step 3, the processing device instructs the user to properly position the ultrasound device on the subject in order to capture clinically usable ultrasound images of a particular anatomical feature (e.g., the heart, bladder, lungs, etc.) and/or from a particular anatomical view (e.g., parasternal long-axis view of the heart, apical four-chamber view of the heart). The instructions to properly position the ultrasound device may include instructions to translate, rotate, and/or tilt the ultrasound device. Further description of examples of instructing a user to properly position an ultrasound device may be found in U. S. Patent No. 10,702,242 titled "AUGMENTED REALITY INTERFACE FOR ASSISTING A USER TO OPERATION AN ULTRASOUND DEVICE," issued on July 7, 2020; U.S. Patent Application No. 16/118,256 titled "METHODS AND APPARATUS FOR COLLECTION OF ULTRASOUND DATA," filed on August 30, 2018 and published as U.S. Patent Publication US 2019-0059851 A1; U.S. Patent Application No. 16/839,020 titled "METHODS AND APPARATUSES FOR COLLECTION OF ULTRASOUND IMAGES," filed on April 2, 2020 and published as U.S. Patent Publication US 2020-0320695 A1; and U.S. Patent Application No. 17/031,283 titled "METHODS AND APPARATUSES FOR PROVIDING FEEDBACK FOR POSITIONING AN ULTRASOUND DEVICE," filed on September 24, 2020 and published as U.S. Patent Publication US 2021-0093298 A1; all of which are assigned to the assignee of the instant application. The process 100 may remain at step 3 until the processing device determines that the ultrasound device now is properly positioned on the subject, in which case the process 100 proceeds to step 4. However, the process 100 may also return to a previous step if the processing device determines that the ultrasound device no longer has sufficient gel (e.g., too much gel has been wiped off the ultrasound device while moving the ultrasound device). In particular, if the processing device determines that the ultrasound device is in contact with the subject but has insufficient gel (state C), the process 100 proceeds back to step 1.

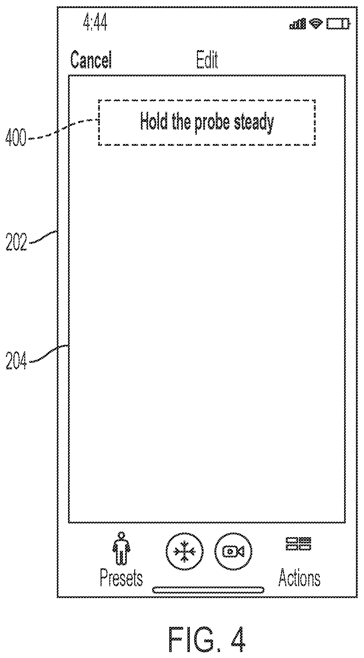

At step 4, the processing device instructs the user to hold the ultrasound device steady. Example instructions are described further with reference to FIG. 4. Because at step 4, the ultrasound device may have sufficient gel and be properly positioned on the subject, the ultrasound device may now collect clinically usable ultrasound images, as long as the user holds the ultrasound device steady. As one particular example, the one or more ultrasound images may constitute a three-dimensional imaging sweep of the bladder, from which the processing device may determine the volume of the bladder. The process 100 may return to step 3 if the processing device determines that the ultrasound device has moved substantially. In some embodiments, the processing device may receive measurements from sensors (e.g., one or more of an accelerometer, gyroscope, and/or magnetometer) on the ultrasound device. If the sensors indicate a change in motion and/or orientation that exceeds a threshold, the processing device may determine that the ultrasound device has moved substantially. Additionally or alternatively, the processing device may determine that the ultrasound device has moved substantially based on determining that the ultrasound device is now in contact with the subject, has sufficient gel, but is no longer properly positioned (i.e., the ultrasound device is in state D). Additionally, the process 100 may return to step 1 if the processing device determines that the ultrasound device no longer has sufficient gel (i.e., the ultrasound device is in state C), for example, because too much gel has been wiped off the ultrasound device while moving the ultrasound device).

For simplicity, certain transitions between steps in FIG. 1 are not illustrated. For example, the process 100 may proceed from steps 3 or 4 to step 2 if the processing device determines that the ultrasound device is in state B. The process 100 may proceed from steps 2, 3 or 4 to step 1 if the processing device determines that the ultrasound device is in state A.

It should be appreciated from the above description that the processing device may provide a current instruction to the user to perform a first action related to preparing the ultrasound device for collecting ultrasound images, and based on detecting a new state of the ultrasound device, automatically transition to providing a new instruction to the user to perform a second action related to preparing the ultrasound device for collecting ultrasound images. Either the first or second action may be any of the instructions described with reference to steps 1, 2, 3, or 4. The new state of the ultrasound device may be any of the states A, B, C, D, or E. The particular new instruction provided may be based on the current instruction provided and the new state of the ultrasound device, as described with reference to FIG. 1.

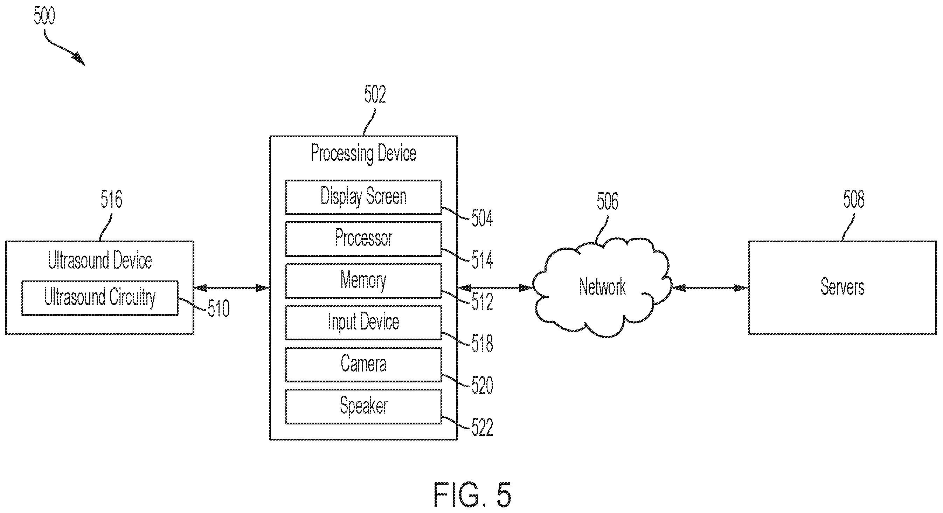

In an embodiment, the steps of process 100 are performed using a single statistical model. The statistical model operates on a processing device such as processing device 502 described further below in connection with FIG. 5. The processing device is a smartphone in some embodiments. The processing device is a tablet computer in some embodiments. The processing device may display a graphical user interface (GUI) of the types shown in FIGs. 2-4 and described further below. In an embodiment, the steps of process 100 are performed usin ga single statistical model having millions of parameters and the application of which involves hundreds of millions of calculations.

FIGs. 2-4 illustrate example graphical user interfaces (GUIs) 200-400 for guiding a user to collect one or more ultrasound images, in accordance with certain embodiments described herein. The GUIs 200-400 are displayed on a display screen 204 of a processing device 202 (e.g., the processing device described with reference to the process 100). It should be appreciated that the exact texts and forms of the GUIs 200-400 are non-limiting. For example, some GUIs may have different texts. Some GUIs may include images and/or videos and/or sound.

The GUI 200 illustrates an instruction to the user to apply gel to the ultrasound device. The GUI 200 may be displayed in conjunction with step 1 of the process 100.

The GUI 300 illustrates an instruction to the user to position the ultrasound device on the subject. The GUI 300 may be displayed in conjunction with step 2 of the process 100. The specific text of GUI 300 may be associated with capturing ultrasound images of the bladder. Examples GUIs illustrating instructions to the user for properly positioning the ultrasound device on the subject in order to capture clinically usable ultrasound images of a particular anatomical feature and/or from a particular anatomical view may be found in U.S. Patent No. 10,702,242, titled "AUGMENTED REALITY INTERFACE FOR ASSISTING A USER TO OPERATION AN ULTRASOUND DEVICE," and issued on July 7, 2020; U.S. Patent Application No. 16/118,256, titled "METHODS AND APPARATUS FOR COLLECTION OF ULTRASOUND DATA," and filed on August 30, 2018; U.S. Patent Application No. 16/839,020, titled "METHODS AND APPARATUSES FOR COLLECTION OF ULTRASOUND IMAGES," and filed on April 2, 2020; and U.S. Patent Application No. 17/031,283, titled "METHODS AND APPARATUSES FOR PROVIDING FEEDBACK FOR POSITIONING AN ULTRASOUND DEVICE," and filed on September 24, 2020.

The GUI 400 illustrates an instruction to the user to hold the ultrasound device steady. The GUI 400 may be displayed in conjunction with step 4 of the process 100.

It should be appreciated that the processing device may cause any of the GUIs described herein, including the GUIs 200-400, to progress automatically from one to another, without requiring the user to specifically select an option to progress from one GUI to another. For example, the processing device may progress from the GUI 200 to the GUI 300 automatically, upon determining that the user has applied sufficient gel to the ultrasound device. As another example, the processing device may progress from the GUI 300 to the GUI 400 automatically, upon determining that the user has properly positioned the ultrasound device on the subject.

FIG. 5 illustrates a schematic block diagram of an example ultrasound system 500 upon which various aspects of the technology described herein may be practiced. The ultrasound system 500 includes an ultrasound device 516, a processing device 502, a network 506, and one or more servers 508. The processing device 502 may be any of the processing devices described herein (e.g., the processing device 202). The ultrasound device 516 may be any of the ultrasound devices described herein.

The ultrasound device 516 includes ultrasound circuitry 510. The processing device 502 includes a camera 520, a display screen 504, a processor 514, a memory 512, an input device 518, and a speaker 522. The processing device 502 is in wired (e.g., through a lightning connector or a mini-USB connector) and/or wireless communication (e.g., using BLUETOOTH, ZIGBEE, and/or WiFi wireless protocols) with the ultrasound device 516. The processing device 502 is in wireless communication with the one or more servers 508 over the network 506.

The ultrasound device 516 may be configured to generate ultrasound data that may be employed to generate an ultrasound image. The ultrasound device 516 may be constructed in any of a variety of ways. In some embodiments, the ultrasound device 516 includes a transmitter that transmits a signal to a transmit beamformer which in turn drives transducer elements within a transducer array to emit pulsed ultrasonic signals into a structure, such as a patient. The pulsed ultrasonic signals may be back-scattered from structures in the body, such as blood cells or muscular tissue, to produce echoes that return to the transducer elements. These echoes may then be converted into electrical signals by the transducer elements and the electrical signals are received by a receiver. The electrical signals representing the received echoes are sent to a receive beamformer that outputs ultrasound data. The ultrasound circuitry 510 may be configured to generate the ultrasound data. The ultrasound circuitry 510 may include one or more ultrasonic transducers monolithically integrated onto a single semiconductor die. The ultrasonic transducers may include, for example, one or more capacitive micromachined ultrasonic transducers (CMUTs), one or more CMOS (complementary metal-oxide-semiconductor) ultrasonic transducers (CUTs), one or more piezoelectric micromachined ultrasonic transducers (PMUTs), and/or one or more other suitable ultrasonic transducer cells. In some embodiments, the ultrasonic transducers may be formed on the same chip as other electronic components in the ultrasound circuitry 510 (e.g., transmit circuitry, receive circuitry, control circuitry, power management circuitry, and processing circuitry) to form a monolithic ultrasound device. The ultrasound device 516 may transmit ultrasound data and/or ultrasound images to the processing device 502 over a wired (e.g., through a lightning connector or a mini-USB connector) and/or wireless (e.g., using BLUETOOTH, ZIGBEE, and/or WiFi wireless protocols) communication link.

Referring now to the processing device 502, the processor 514 may include specially-programmed and/or special-purpose hardware such as an application-specific integrated circuit (ASIC). For example, the processor 514 may include one or more graphics processing units (GPUs) and/or one or more tensor processing units (TPUs). TPUs may be ASICs specifically designed for machine learning (e.g., deep learning). The TPUs may be employed, for example, to accelerate the inference phase of a neural network. The processing device 502 may be configured to process the ultrasound data received from the ultrasound device 516 to generate ultrasound images for display on the display screen 504 (of which the display screen 304 may be an example). The processing may be performed by, for example, the processor 514. The processor 514 may also be adapted to control the acquisition of ultrasound data with the ultrasound device 516. The ultrasound data may be processed in real-time during a scanning session as the echo signals are received. In some embodiments, the displayed ultrasound image may be updated a rate of at least 5Hz, at least 10 Hz, at least 20Hz, at a rate between 5 and 60 Hz, at a rate of more than 20 Hz. For example, ultrasound data may be acquired even as images are being generated based on previously acquired data and while a live ultrasound image is being displayed. As additional ultrasound data is acquired, additional frames or images generated from more-recently acquired ultrasound data may be sequentially displayed. Additionally, or alternatively, the ultrasound data may be stored temporarily in a buffer during a scanning session and processed in less than real-time.

The processing device 502 may be configured to perform certain of the processes (e.g., the process 100) described herein using the processor 514 (e.g., one or more computer hardware processors) and one or more articles of manufacture that include non-transitory computer-readable storage media such as the memory 512. The processor 514 may control writing data to and reading data from the memory 512 in any suitable manner. To perform certain of the processes described herein, the processor 514 may execute one or more processor-executable instructions stored in one or more non-transitory computer-readable storage media (e.g., the memory 512), which may serve as non-transitory computer-readable storage media storing processor-executable instructions for execution by the processor 514. The camera 520 may be configured to detect light (e.g., visible light) to form an image. The camera 520 may be on the same face of the processing device 502 as the display screen 504. The display screen 504 may be configured to display images and/or videos, and may be, for example, a liquid crystal display (LCD), a plasma display, and/or an organic light emitting diode (OLED) display on the processing device 502. The input device 518 may include one or more devices capable of receiving input from a user and transmitting the input to the processor 514. For example, the input device 518 may include a keyboard, a mouse, a microphone, touch-enabled sensors on the display screen 504, and/or a microphone. The display screen 504, the input device 518, the camera 520, and the speaker 522 may be communicatively coupled to the processor 514 and/or under the control of the processor 514.

It should be appreciated that the processing device 502 may be implemented in any of a variety of ways. For example, the processing device 502 may be implemented as a handheld device such as a mobile smartphone or a tablet. Thereby, a user of the ultrasound device 516 may be able to operate the ultrasound device 516 with one hand and hold the processing device 502 with another hand. In other examples, the processing device 502 may be implemented as a portable device that is not a handheld device, such as a laptop. In yet other examples, the processing device 502 may be implemented as a stationary device such as a desktop computer. The processing device 502 may be connected to the network 506 over a wired connection (e.g., via an Ethernet cable) and/or a wireless connection (e.g., over a WiFi network). The processing device 502 may thereby communicate with (e.g., transmit data to or receive data from) the one or more servers 508 over the network 506. For example, a party may provide from the server 508 to the processing device 502 processor-executable instructions for storing in one or more non-transitory computer-readable storage media (e.g., the memory 512) which, when executed, may cause the processing device 502 to perform certain of the processes (e.g., the process 100) described herein.

Various embodiments described herein utilize statistical models to perform one or more functions. It should be appreciated that the statistical models may include tens of thousands, hundreds of thousands, or millions of parameters. For example, the statistical models described in connection with FIG. 1 may include millions of parameters. For example, the statistical model used in performance of the process 100 includes between 200,000 and 20,000,000 parameters, including any range of parameters within that stated range. Applying the statistical model(s) described herein requires many calculations, which cannot be done practically in the human mind and without computers. For example, applying the statistical model(s) when performing one or more steps of the process 100 involves performing millions of calculations in some embodiments. In some embodiments, hundreds of millions or billions of calculations are performed. Any such statistical models are trained with tens, hundreds, or thousands of ultrasound images. Neither training nor using the statistical model(s) may be accomplished without computing resources.

Various aspects of the present disclosure may be used alone, in combination, or in a variety of arrangements not specifically described in the embodiments described in the foregoing and is therefore not limited in its application to the details and arrangement of components set forth in the foregoing description or illustrated in the drawings. For example, aspects described in one embodiment may be combined in any manner with aspects described in other embodiments.

Various inventive concepts may be embodied as one or more processes, of which an example has been provided. The acts performed as part of each process may be ordered in any suitable way. Thus, embodiments may be constructed in which acts are performed in an order different than illustrated, which may include performing some acts simultaneously, even though shown as sequential acts in illustrative embodiments. Further, one or more of the processes may be combined and/or omitted, and one or more of the processes may include additional steps.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

As used herein, reference to a numerical value being between two endpoints should be understood to encompass the situation in which the numerical value can assume either of the endpoints. For example, stating that a characteristic has a value between A and B, or between approximately A and B, should be understood to mean that the indicated range is inclusive of the endpoints A and B unless otherwise noted.

Also, the phraseology and terminology used herein is for the purpose of description and should not be regarded as limiting. The use of "including," "comprising," or "having," "containing," "involving," and variations thereof herein, is meant to encompass the items listed thereafter as well as additional items.

Having described above several aspects of at least one embodiment, it is to be appreciated various alterations, modifications, and improvements will readily occur to those skilled in the art. Such alterations, modifications, and improvements are intended to be object of this disclosure. Accordingly, the foregoing description and drawings are by way of example only.

## Claims

1. An apparatus, comprising:
a processing device (502) in operative communication with an ultrasound device (516), the processing device (502) configured to:
determine that sufficient ultrasound coupling medium has not been applied to the ultrasound device; and
based on determining that sufficient ultrasound coupling medium has not been applied to the ultrasound device (516), instruct a user to apply sufficient ultrasound coupling medium to the ultrasound device (516), wherein the processing device (516) is configured to determine that sufficient ultrasound coupling medium has not been applied to the ultrasound device (516) using a statistical model trained on ultrasound images collected with and labeled as having been collected with no gel, low amounts of gel, medium amounts of gel, and high amounts of gel applied to the ultrasound device (516);
wherein the processing device (502) is further configured to determine a state of the ultrasound device (516) based on ultrasound images collected by the ultrasound device; and
wherein the processing device (502) is configured, when instructing the user to apply sufficient ultrasound coupling medium to the ultrasound device (516), to indicate one of multiple levels of ultrasound coupling medium that has been applied to the ultrasound device (516) and/or one of multiple levels of ultrasound coupling medium that still needs to be applied to the ultrasound device (516), the one of multiple levels of ultrasound coupling medium including no gel, low amounts of gel, medium amounts of gel, and high amounts of gel applied to the ultrasound device (516).

2. The apparatus of claim 1, wherein the processing device (502) is configured to receive ultrasound images collected by the ultrasound device (516) while the processing device (502) is determining that sufficient ultrasound coupling medium has not been applied to the ultrasound device (516) and/or while instructing the user to apply sufficient ultrasound coupling medium to the ultrasound device (516).

3. The apparatus of claim 1, wherein the processing device (502) is further configured to: automatically transition to further instruct the user to position the ultrasound device (516) on a subject as a further action in preparing the ultrasound device (516) for collecting ultrasound images based on detecting a new state of the ultrasound device (516); and
wherein the new state of the ultrasound device (516) comprises the ultrasound device having sufficient ultrasound coupling medium applied to it but not being properly positioned.

4. The apparatus of claim 3, wherein the new state of the ultrasound device (516) comprises the ultrasound device (516) having sufficient ultrasound coupling medium applied to it but not being positioned on a subject.

5. The apparatus of claim 3, wherein the further action comprises positioning the ultrasound device (516) on the subject to capture ultrasound images of a particular anatomical feature and/or from a particular anatomical view, and wherein the new state of the ultrasound device (516) comprises the ultrasound device (516) having sufficient ultrasound coupling medium applied to it and being positioned on the subject, but not being properly positioned on the subject in order to capture ultrasound images of the particular anatomical feature and/or from the particular anatomical view.

6. The apparatus of claim 5, wherein the particular anatomical feature comprises a bladder.

7. The apparatus of claim 3, wherein the processing device (502) is configured to detect the new state of the ultrasound device (516) based on ultrasound images more recently collected by the ultrasound device (516).

8. The apparatus of claim 3, wherein the processing device (502) is configured to receive ultrasound images collected by the ultrasound device (516) while the processing device is providing the instruction to apply sufficient ultrasound coupling medium and/or the further instruction to position the ultrasound device (516) on a subject.

9. The apparatus of claim 4, wherein the processing device (502) is configured to determine that the ultrasound device (516) is not positioned on the subject using the statistical model.

10. The apparatus of claim 4, wherein the processing device (502) is configured to determine that the ultrasound device (516) is not positioned on the subject based on ultrasound images more recently collected by the ultrasound device (516).

11. The apparatus of claim 4, wherein the processing device (502) is configured to receive ultrasound images collected by the ultrasound device (516) while the processing device (502) is determining that the ultrasound device (516) is not positioned on the subject and/or while instructing the user to position the ultrasound device (516) on the subject.

12. The apparatus of claim 1, wherein the processor device (502) is further configured to: instruct a user to hold the ultrasound device (516) steady;
determine that the user has not held the ultrasound device (516) steady; and
based on determining that the user has not held the ultrasound device (516) steady, instruct the user to properly position the ultrasound device (516) on the subject in order to capture ultrasound images of a particular anatomical feature and/or from the particular anatomical view.

13. The apparatus of claim 12, wherein the processing device (502) is configured to determine that the user has not held the ultrasound device (516) steady using the statistical model.

## Patentansprüche

1. Vorrichtung, umfassend:
eine Verarbeitungsvorrichtung (502), die in funktionaler Verbindung mit einer Ultraschallvorrichtung (516) steht, wobei die Verarbeitungsvorrichtung (502) so konfiguriert ist, dass sie:
feststellen, dass dem Ultraschallgerät nicht ausreichend Ultraschall-Kopplungsmedium zugeführt wurde; und
auf der Grundlage der Feststellung, dass nicht ausreichend Ultraschall-Kopplungsmittel auf das Ultraschallgerät (516) aufgetragen wurde, den Benutzer anzuweisen, ausreichend Ultraschall-Kopplungsmittel auf das Ultraschallgerät (516) aufzutragen, wobei das Verarbeitungsgerät (516) so konfiguriert ist, dass es feststellt, dass kein ausreichendes Ultraschall-Kopplungsmittel auf das Ultraschallgerät (516) aufgetragen wurde, und zwar unter Verwendung eines statistischen Modells, das anhand von Ultraschallbildern trainiert wurde, die mit und als "ohne Gel", "geringe Gelmenge", "mittlere Gelmenge" und "hohe Gelmenge" gekennzeichnet aufgenommen wurden, wobei diese Mengen an Gel auf das Ultraschallgerät (516) aufgetragen wurden;
wobei die Verarbeitungseinrichtung (502) ferner so konfiguriert ist, dass sie den Zustand des Ultraschallgeräts (516) anhand der vom Ultraschallgerät erfassten Ultraschallbilder bestimmt; und
wobei die Verarbeitungsvorrichtung (502) so konfiguriert ist, dass sie beim Aufbringen einer ausreichenden Menge Ultraschall-Kopplungsmedium auf das Ultraschallgerät (516) durch den Benutzer eine der mehreren Stufen des bereits auf das Ultraschallgerät (516) aufgebrachten Ultraschall-Kopplungsmediums und/oder eine der mehreren Stufen des noch auf das Ultraschallgerät (516) aufzutragenden Ultraschall-Kopplungsmediums anzeigt, wobei die eine der mehreren Stufen des Ultraschall-Kopplungsmediums kein Gel, geringe Gelmengen, mittlere Gelmengen und hohe Gelmengen umfasst, die auf das Ultraschallgerät (516) aufgebracht wurden.

2. Die Vorrichtung nach Anspruch 1, wobei die Verarbeitungseinrichtung (502) so konfiguriert ist, dass sie vom Ultraschallgerät (516) erfasste Ultraschallbilder empfängt, während die Verarbeitungseinrichtung (502) feststellt, dass nicht ausreichend Ultraschallkopplungsmedium auf das Ultraschallgerät (516) aufgebracht wurde, und/oder während sie den Benutzer anweist, ausreichend Ultraschallkopplungsmedium auf das Ultraschallgerät (516) aufzubringen.

3. Die Vorrichtung nach Anspruch 1, wobei die Verarbeitungsvorrichtung (502) ferner so ausgelegt ist, dass sie:
automatisch zum nächsten Schritt übergehen, um den Benutzer anzuweisen, das Ultraschallgerät (516) auf dem Probanden zu positionieren, als weitere Maßnahme zur Vorbereitung des Ultraschallgeräts (516) auf die Aufnahme von Ultraschallbildern, basierend auf der Erkennung eines neuen Zustands des Ultraschallgeräts (516); und
wobei der neue Zustand der Ultraschallvorrichtung (516) darin besteht, dass auf die Ultraschallvorrichtung ausreichend Ultraschall-Koppelmedium aufgebracht ist, diese jedoch nicht ordnungsgemäß positioniert ist.

4. Die Vorrichtung nach Anspruch 3, wobei sich die Ultraschallvorrichtung (516) im neuen Zustand darin befindet, dass ausreichend Ultraschall-Koppelmedium auf die Ultraschallvorrichtung (516) aufgebracht ist, diese aber nicht auf einem Objekt positioniert ist.

5. Die Vorrichtung nach Anspruch 3, wobei die weitere Maßnahme darin besteht, das Ultraschallgerät (516) auf dem Subjekt zu positionieren, um Ultraschallbilder eines bestimmten anatomischen Merkmals und/oder aus einer bestimmten anatomischen Ansicht aufzunehmen, und wobei der neue Zustand des Ultraschallgeräts (516) darin besteht, dass auf das Ultraschallgerät (516) ausreichend Ultraschall-Koppelmedium aufgebracht ist und es auf dem Subjekt positioniert ist, jedoch nicht richtig auf dem Subjekt positioniert ist, um Ultraschallbilder des bestimmten anatomischen Merkmals und/oder aus der bestimmten anatomischen Ansicht aufzunehmen.

6. Die Vorrichtung nach Anspruch 5, wobei das besondere anatomische Merkmal eine Blase umfasst.

7. Die Vorrichtung nach Anspruch 3, wobei die Verarbeitungseinrichtung (502) so konfiguriert ist, dass sie den neuen Zustand des Ultraschallgeräts (516) anhand von Ultraschallbildern erkennt, die zuletzt vom Ultraschallgerät (516) aufgenommen wurden.

8. Die Vorrichtung nach Anspruch 3, wobei die Verarbeitungseinrichtung (502) so konfiguriert ist, dass sie Ultraschallbilder empfängt, die von dem Ultraschallgerät (516) erfasst wurden, während die Verarbeitungseinrichtung die Anweisung gibt, ausreichend Ultraschall-Koppelmedium aufzutragen und/oder die weitere Anweisung, das Ultraschallgerät (516) auf einem Subjekt zu positionieren.

9. Die Vorrichtung nach Anspruch 4, wobei die Verarbeitungseinrichtung (502) so konfiguriert ist, dass sie mithilfe des statistischen Modells feststellt, dass sich das Ultraschallgerät (516) nicht auf dem Subjekt befindet.

10. Die Vorrichtung nach Anspruch 4, wobei die Verarbeitungsvorrichtung (502) so konfiguriert ist, dass sie auf der Grundlage von Ultraschallbildern, die kürzlich von der Ultraschallvorrichtung (516) aufgenommen wurden, feststellt, dass die Ultraschallvorrichtung (516) nicht am Untersuchungsobjekt positioniert ist.

11. Die Vorrichtung nach Anspruch 4, wobei die Verarbeitungseinrichtung (502) so konfiguriert ist, dass sie Ultraschallbilder empfängt, die von dem Ultraschallgerät (516) aufgenommen wurden, während die Verarbeitungseinrichtung (502) feststellt, dass sich das Ultraschallgerät (516) nicht auf dem Subjekt befindet, und/oder während sie den Benutzer anweist, das Ultraschallgerät (516) auf dem Subjekt zu positionieren.

12. Die Vorrichtung nach Anspruch 1, wobei die Prozessorvorrichtung (502) ferner so konfiguriert ist, dass sie den Benutzer anweist, das Ultraschallgerät (516) ruhig zu halten;
feststellen, dass der Benutzer das Ultraschallgerät (516) nicht ruhig gehalten hat; und
Wenn festgestellt wird, dass der Benutzer das Ultraschallgerät (516) nicht ruhig hält, weisen Sie den Benutzer an, das Ultraschallgerät (516) richtig auf dem Patienten zu positionieren, um Ultraschallbilder eines bestimmten anatomischen Merkmals und/oder aus der jeweiligen anatomischen Ansicht aufzunehmen.

13. Die Vorrichtung nach Anspruch 12, wobei die Verarbeitungseinrichtung (502) so konfiguriert ist, dass sie mithilfe des statistischen Modells feststellt, ob der Benutzer das Ultraschallgerät (516) nicht ruhig gehalten hat.

## Revendications

1. Appareil, comprenant :
un dispositif de traitement (502) en communication opérationnelle avec un dispositif ultrasonore (516), le dispositif de traitement (502) étant configuré pour :
déterminer qu'un milieu de couplage ultrasonore suffisant n'a pas été appliqué au dispositif ultrasonore ; et
sur la base de la détermination selon laquelle le milieu de couplage ultrasonore suffisant n'a pas été appliqué au dispositif ultrasonore (516), ordonner à un utilisateur d'appliquer un milieu de couplage ultrasonore suffisant au dispositif ultrasonore (516), dans lequel le dispositif de traitement (516) est configuré pour déterminer que le milieu de couplage ultrasonore suffisant n'a pas été appliqué au dispositif ultrasonore (516) à l'aide d'un modèle statistique entraîné sur des images ultrasonores collectées et étiquetées comme ayant été collectées sans gel, avec de faibles quantités de gel, des quantités moyennes de gel et des quantités élevées de gel appliquées au dispositif ultrasonore (516) ;
dans lequel le dispositif de traitement (502) est en outre configuré pour déterminer un état du dispositif ultrasonore (516) sur la base d'images ultrasonores collectées par le dispositif ultrasonore ; et
dans lequel le dispositif de traitement (502) est configuré, lorsqu'il ordonne à l'utilisateur d'appliquer un milieu de couplage ultrasonore suffisant au dispositif ultrasonore (516), pour indiquer l'un des multiples niveaux de milieu de couplage ultrasonore qui a été appliqué au dispositif ultrasonore (516) et/ou l'un des multiples niveaux de milieu de couplage ultrasonore qui doit encore être appliqué au dispositif ultrasonore (516), l'un des multiples niveaux de milieu de couplage ultrasonore comportant aucun gel, de faibles quantités de gel, des quantités moyennes de gel et des quantités élevées de gel appliquées au dispositif ultrasonore (516).

2. Appareil selon la revendication 1, dans lequel le dispositif de traitement (502) est configuré pour recevoir des images ultrasonores collectées par le dispositif ultrasonore (516) pendant que le dispositif de traitement (502) détermine qu'un milieu de couplage ultrasonore suffisant n'a pas été appliqué au dispositif ultrasonore (516) et/ou pendant qu'il ordonne à l'utilisateur d'appliquer un milieu de couplage ultrasonore suffisant au dispositif ultrasonore (516).

3. Appareil selon la revendication 1, dans lequel le dispositif de traitement (502) est en outre configuré pour :
passer automatiquement à l'étape consistant à ordonner en outre à l'utilisateur de positionner le dispositif ultrasonore (516) sur un sujet, en tant qu'action supplémentaire de préparation du dispositif ultrasonore (516) pour la collecte d'images ultrasonores, sur la base de la détection d'un nouvel état du dispositif ultrasonore (516) ; et
dans lequel le nouvel état du dispositif ultrasonore (516) comprend le fait qu'un milieu de couplage ultrasonore suffisant est appliqué au dispositif ultrasonore mais que ce dernier n'est pas correctement positionné.

4. Appareil selon la revendication 3, dans lequel le nouvel état du dispositif ultrasonore (516) comprend le fait qu'un milieu de couplage ultrasonore suffisant est appliqué au dispositif ultrasonore (516) mais que ce dernier n'est pas correctement positionné sur un sujet.

5. Appareil selon la revendication 3, dans lequel l'action supplémentaire comprend le positionnement du dispositif ultrasonore (516) sur le sujet pour capturer des images ultrasonores d'une caractéristique anatomique particulière et/ou d'une vue anatomique particulière, et dans lequel le nouvel état du dispositif ultrasonore (516) comprend le fait qu'un milieu de couplage ultrasonore suffisant est appliqué au dispositif ultrasonore (516) et que ce dernier est positionné sur le sujet, mais qu'il n'est pas correctement positionné sur le sujet pour pouvoir capturer des images ultrasonores de la caractéristique anatomique particulière et/ou de la vue anatomique particulière.

6. Appareil selon la revendication 5, dans lequel la caractéristique anatomique particulière comprend une vessie.

7. Appareil selon la revendication 3, dans lequel le dispositif de traitement (502) est configuré pour détecter le nouvel état du dispositif ultrasonore (516) sur la base d'images ultrasonores collectées en dernier par le dispositif ultrasonore (516).

8. Appareil selon la revendication 3, dans lequel le dispositif de traitement (502) est configuré pour recevoir des images ultrasonores collectées par le dispositif ultrasonore (516) pendant que le dispositif de traitement donne l'ordre d'appliquer un milieu de couplage ultrasonore suffisant et/ou l'ordre ultérieur de positionner le dispositif ultrasonore (516) sur un sujet.

9. Appareil selon la revendication 4, dans lequel le dispositif de traitement (502) est configuré pour déterminer que le dispositif ultrasonore (516) n'est pas positionné sur le sujet à l'aide du modèle statistique.

10. Appareil selon la revendication 4, dans lequel le dispositif de traitement (502) est configuré pour déterminer que le dispositif ultrasonore (516) n'est pas positionné sur le sujet sur la base d'images ultrasonores collectées en dernier par le dispositif ultrasonore (516).

11. Appareil selon la revendication 4, dans lequel le dispositif de traitement (502) est configuré pour recevoir des images ultrasonores collectées par le dispositif ultrasonore (516) pendant que le dispositif de traitement (502) détermine que le dispositif ultrasonore (516) n'est pas positionné sur le sujet et/ou pendant qu'il ordonne à l'utilisateur de positionner le dispositif ultrasonore (516) sur le sujet.

12. Appareil selon la revendication 1, dans lequel le dispositif de traitement (502) est en outre configuré pour : ordonner à un utilisateur de maintenir le dispositif ultrasonore (516) stable ;
déterminer que l'utilisateur n'a pas maintenu le dispositif ultrasonore (516) stable ; et
sur la base de la détermination selon laquelle l'utilisateur n'a pas maintenu le dispositif ultrasonore (516) stable, ordonner à l'utilisateur de positionner correctement le dispositif ultrasonore (516) sur le sujet pour pouvoir capturer des images ultrasonores d'une caractéristique anatomique particulière et/ou d'une vue anatomique particulière.

13. Appareil selon la revendication 12, dans lequel le dispositif de traitement (502) est configuré pour déterminer que l'utilisateur n'a pas maintenu le dispositif ultrasonore (516) stable à l'aide du modèle statistique.
